# EUROPEAN PATENT APPLICATION

(11) **EP 4 403 643 A1**
(43) Date of publication of application: **24.07.2024**
(21) Application number: 23152484.4
(22) Date of filing: 19.01.2023
(51) Int. Cl.: C12Q 1/6806, C12Q 1/6834, C12Q 1/6874

(54) **MULTIPLE MOLECULAR BINDING SITES**

(71) Applicant: Gnothis Holding AG, 6330 Cham (CH)
(72) Inventor: EDMAN, Lars, 619 92 Trosa (SE); WIND, Samuel (Shalom) J., New York, 10605 (US)
(74) Representative: Weickmann & Weickmann PartmbB

(57) **Abstract**

The present disclosure relates to supports comprising a substrate and a plurality of sample spots on the surface of the support, wherein the sample spots are spatially separated from another by the substrate, and wherein a biomolecule is immobilized on at least 2 separate sample spots.

## Description

The present disclosure relates to supports comprising a substrate and a plurality of sample spots on the surface of the support, wherein the sample spots are spatially separated from another, and wherein a biomolecule is immobilized on at least two separate sample spots.

### Background

Sequencing of the human genome or the genome of other organisms and the determination and comparison of individual sequence variants requires the provision of sequencing methods which firstly are fast and secondly can be employed routinely and cost-effectively.

The high demand for cost-efficient sequencing has driven the development of high-throughput sequencing technologies that parallelize the sequencing process producing a plurality of sequences concurrently. Examples of these sequencing technologies are massively parallel signature sequencing (Lynx Therapeutics), polony sequencing (Life Technologies), 454 pyrosequencing (Roche Diagnostics), illumina sequencing (Solexa Inc.), sequencing by ligation (Life Technologies), ion torrent semiconductor sequencing (Life Technologies) or DNA nanoball sequencing (Complete Genomics). These technologies allow rapid analysis of a consensus sequence in a nucleic acid population. Mutations existing in minority sequences in the nucleic acid population to be analysed, e.g., in a minority of cellular genomes, however, will not be detected since they are obscured by the majority of other sequences present in the population.

In order to address this issue, single molecule sequencing processes in several different formats have been developed. Typically, nucleic acid-polymerizing enzymes and/or nucleic acid-degrading enzymes and fluorescence-labelled nucleic acids and/or nucleotide building blocks are used for individually determining the sequence of a single nucleic acid molecule on the basis of a time-dependent change in fluorescence when nucleotide building blocks are incorporated into or cleaved off from a nucleic acid molecule. Single molecule sequencing processes and devices adapted for performing such processes are e.g., described in co-owned applications WO 2002/097406, WO 2003/052137, WO 2006/013110, WO 2013/131888, WO 2015/104245, WO 2017/001407, and WO 2018/104301.

In several of these processes, nucleic acid-degrading enzyme molecules and/or nucleic acid-synthesizing enzyme molecules or complexes of such enzyme molecules with nucleic acid molecules are provided in an immobilized form on a solid support.

US 7,745,116 B2, the content of which is herein incorporated by reference, discloses compositions and methods for single molecule sequencing by detecting incorporation of a labelled nucleoside triphosphate onto the growing end of a primer molecule. In particular, a polymerase-nucleic acid complex comprising at least one anchor, e.g., two anchors, is provided. The anchor may be selected from an amino acid, an epitope, a modified amino acid, a histidine tag etc. The anchor may be used for attaching a topological tether to the polymerase, or for attaching the polymerase to a support. The attachment of a single polymerase molecule to multiple separate spots on a substrate is not disclosed.

It was an object of the present disclosure to provide supports comprising immobilized biomolecules including biomolecule complexes in precisely defined and/or favourable steric configurations suitable for an analysis of single molecule events, e.g., for a sequence analysis of a single nucleic acid molecule.

### Summary of the Invention

In a first aspect, the present disclosure relates to a support comprising a substrate and a plurality of sample spots on the surface of the support, wherein the sample spots are spatially separated from another, and wherein a single biomolecule is immobilized on at least two separate sample spots. In a particular embodiment, the substrate is an optically transparent substrate.

A further aspect of the present disclosure relates to the use of the above support for analyzing an event, i.e., one or several events, at the at least two sample spots wherein said event is associated with emission of an electromagnetic radiation. In a particular embodiment, the event is a single molecule event. In certain embodiments, analyzing an event at the at least two sample spots also includes analyzing an event, i.e., one or several events between the at least two sample spots.

A further aspect relates to a method for analyzing an event, e.g., a single molecule event comprising:
(i) providing the above support, wherein a single biomolecule is immobilized on at least two separate sample spots of the support, and
(ii) analyzing an event associated with said biomolecule, particularly a single molecule event associated with said single biomolecule, by detecting electromagnetic radiation from said sample spots.

In certain embodiments, analyzing an event associated with the biomolecule by detecting electromagnetic radiation from the at least two sample spots also includes detecting electromagnetic radiation from an area on the support between the at least two sample spots.

In a particular embodiment, the single molecule event comprises a sequence analysis of a single nucleic acid molecule.

A further aspect relates to a device adapted for analyzing an event, e.g., a single molecule event comprising:
(i) the above support, wherein a single biomolecule is immobilized on at least two separate sample spots of the support,
(ii) means for illuminating the at least two separate sample spots on the support with radiation, and
(iii) means for analyzing an event on said at least two sample spots by detecting electromagnetic radiation from said sample spots.

In certain embodiments, means for analyzing an event on the at least two sample spots associated with the biomolecule by detecting electromagnetic radiation from the at least two sample spots also includes means for detecting electromagnetic radiation from an area on the support between the at least two sample spots.

In a particular embodiment, the device is adapted for the sequence analysis of a single nucleic acid molecule.

### Items of the Specification

1. A support comprising a substrate and a plurality of sample spots on the surface of the support, wherein the sample spots are spatially separated from another, and wherein a biomolecule is immobilized on at least 2 separate sample spots.
2. The support of item 1, wherein the biomolecule is immobilized on 2, 3 or 4 separate sample spots, particularly on 2 separate sample spots.
3. The support of item 1 or 2, which is at least substantially planar.
4. The support of item 1 or 2, which is structured.
5. The support of any one of the preceding items, wherein the substrate is optically transparent.
6. The support of any one of the preceding items, wherein the substrate comprises a material having a refractive index of at least 1.01.
7. The support of any one of the preceding items, wherein the substrate comprises a non-electrically conductive material.
8. The support of any one of the preceding items, wherein the substrate comprises a material selected from the group consisting of silica, quartz, and glass.
9. The support of any one of the preceding items, wherein the substrate has a thickness of about 10 µm to about 5 mm, particularly about 20 µm to about 2 mm.
10. The support of any one of the preceding items, which comprises at least 10, at least 100, at least 1,000, at least 10,000, at least 100,000, at least 1,000,000 or at least 10,000,000 sample spots.
11. The support of any one of the preceding items, wherein the at least one sample spot comprises at least one electrically conductive material, e.g., a metal including a pure metal or a combination of metals, e.g., an alloy or mixture of a plurality of different metals.
12. The support of item 11, wherein the at least one metal is capable of forming a bond with sulfur, e.g., in the form of a thiol or a disulfide.
13. The support of any one of items 11-12, wherein the at least metal has a positive electrochemical potential.
14. The support of any one of items 11-13, wherein the at least one metal is selected from Au, Cu, Ni, Pt, Pd, Rh, Ir, Os, Ru and any combination comprising at least two of said metals.
15. The support of any one of items 1-10, wherein the at least one sample spot comprises at least one metal oxide such as TiO₂, NiO, or ITO.
16. The support of any one of the preceding items, wherein the at least one sample spot has a diameter of about 1 nm to about 30 nm, particularly a diameter of about 2 nm to about 20 nm.
17. The support of any one of the preceding items, wherein the sample spots are distributed substantially evenly on the support surface.
18. The support of item 17, wherein the distance between adjacent sample spots is about 1 nm to about 50 nm, e.g., about 2 nm to about 20 nm.
19. The support of any one of the preceding items, wherein the sample spots are distributed unevenly on the support surface.
20. The support of item 19, wherein the sample spots are distributed in groups of several spots, e.g., about 2 to about 10 spots or about 2 to about 4 spots on the support surface, wherein the distance between adjacent sample spots in different groups within a group is greater than the distance between adjacent sample spots in different groups.
21. The support of item 20, wherein the distance between adjacent sample spots within a group is about 1 nm to about 500 nm, e.g., about 2 nm to about 20 nm, or about 20 nm to about 500 nm.
22. The support of item 20 or 21, wherein the distance between adjacent sample spots in different groups is about 2- to about 2000-times greater, e.g., about 5- to about 1000-times or about 10- to about 100-times greater than the distance between adjacent sample spots in the same group.
23. The support of any one of the preceding items, wherein the biomolecule is selected from the group consisting of polypeptides, nucleic acids, carbohydrates, and any combination thereof.
24. The support of any one of the preceding items, wherein the biomolecule is a nucleic acid-polymerizing enzyme, particularly a DNA polymerase or an RNA-polymerase, or a nucleic acid-polymerizing molecule complex, particularly a DNA- or RNA-polymerizing complex comprising a nucleic acid-polymerizing enzyme and a nucleic acid molecule.
25. The support of item 24, wherein the biomolecule is a DNA polymerase having a DNA binding cleft, particularly a Family A DNA polymerase including, but not limited to a Klenow, Taq or T7 DNA polymerase or any genetically modified version thereof, or a Family B polymerase including, but not limited to a therminator, Phi29, RB-69 or T4 DNA polymerase or any genetically modified version thereof.
26. The support of any one of items 1-23, wherein the biomolecule is a gene editing enzyme, particularly a Cas nuclease such as a Cas9 nuclease or any genetically modified version thereof, or a gene editing complex comprising a gene editing enzyme and nucleic acid molecule, e.g., a guide RNA and/or a target nucleic acid.
27. The support of any one of items 1-23, wherein the biomolecule is a nucleic acid molecule, e.g., a single- or double-stranded nucleic acid molecule, particularly DNA molecule or an RNA molecule.
28. The support of item 27, wherein the biomolecule is a single- stranded DNA or RNA molecule.
29. The support of item 27 or 28, wherein the nucleic acid molecule has a length of at least about 10 nucleotides, at least about 100 nucleotides, at least about 1,000 nucleotides, e.g., about 1,000 to about 100,000, particularly about 10,000 to about 50,000 nucleotides.
30. The support of any one of the preceding items, wherein the biomolecule comprises at least 2 anchors attached to at least 2 separate sample spots.
31. The support of item 30, wherein the anchor comprises the reaction product between a sulfur-containing group or a phosphorus-containing group and a metal or metal oxide surface on the sample spots, the reaction product between a reactive silane group with a metal oxide surface on the sample spots, or the reaction product of a poly(histidine) tag with a Ni or NiO surface on the sample spots.
32. The support of item 30, wherein the anchor comprises an attachment amino acid or an attachment tag such as a biotin, hapten, poly(histidine)tag or carbohydrate group, bound to a complementary moiety of a coating attached to the surface of the sample spots such as streptavidin, antibody, or lectin.
33. The support of item 32, wherein the at least one attachment amino acid is selected from cysteine, a modified phenylalanine, histidine, and glutamine.
34. The support of item 30, wherein the anchor comprises the reaction product of 2 bio-orthogonal groups, e.g., an azide group or an alkyne group.
35. The support of item 34, wherein the anchor comprises a triazole group.
36. Use of a support of any one of items 1-35 for analyzing an event occurring on the at least two separate sample spots, wherein said event is associated with emission of an electromagnetic radiation.
37. The use of item 36 for analyzing one or several events occurring on the at least two separate sample spots and/or between the at least two sample spots.
38. Use of a support of any one of items 1-35 for analyzing a single molecule event occurring on the at least two separate sample spots, wherein said single molecule event is associated with emission of an electromagnetic radiation.
39. The use of item 38 for analyzing one or several events occurring on the at least two separate sample spots and/or between the at least two sample spots.
40. Use of a support of any one of items 1-35 for separately analyzing a plurality of events, particularly single molecule events each occurring on at least two separate sample spots, wherein said events are associated with emission of an electromagnetic radiation.
41. The use of item 40 for analyzing a plurality of events each occurring on the at least two separate sample spots and/or between the at least two sample spots.
42. The use of item 40 or 41 wherein a plurality of events, particularly single molecule events is analyzed in parallel.
43. The use of any one of items 36-42 wherein the single molecule event comprises a nucleic acid sequence determination.
44. The use of item 43, wherein the nucleic acid sequence determination comprises
   - providing (i) a single nucleic acid molecule, (ii) a nucleic acid-synthesizing enzyme molecule and/or a nucleic acid degrading enzyme molecule, immobilized on at least two separate sample spots and (iii) fluorescence labelled nucleotide building blocks in free form and/or incorporated into the nucleic acid molecule,
   - conducting an enzymatic reaction, wherein nucleotide building blocks are incorporated into and/or cleaved off from said single nucleic acid molecule, and
   - individually determining the base sequence of the nucleic acid molecule on the basis of the time-dependent fluorescence change, caused when nucleotide building blocks are incorporated into and/or cleaved off from said single nucleic acid molecule.
45. The use of item 44, wherein a nucleic acid-synthesizing enzyme molecule and/or a nucleic acid degrading enzyme molecule is immobilized on said sample spots.
46. The use of item 44, wherein a single nucleic acid molecule is immobilized on said sample spots.
47. A method for analyzing an event, e.g., a single molecule event comprising:
   (iii) providing a support of any one of items 1-35, wherein a single biomolecule is immobilized on at least two separate sample spots of the support, and
   (iv) analyzing an event, associated with said biomolecule, particularly a single molecule event associated with said single biomolecule, by detecting electromagnetic radiation from said sample spots.
48. The method of item 47, wherein analyzing an event associated with the biomolecule by detecting electromagnetic radiation from the at least two sample spots includes detecting electromagnetic radiation from an area on the support between the at least two sample spots.
49. The method of item 47 or 48 wherein the single molecule event comprises a sequence analysis of a single nucleic acid molecule.
50. A device for analyzing an event, e.g., a single molecule event comprising:
   (iv) a support of any one of items 1-35, wherein a single biomolecule is immobilized on at least two separate sample spots of the support,
   (v) means for illuminating the at least two separate sample spots on the support with radiation, and
   (vi) means for analyzing an event on said at least two sample spots by detecting electromagnetic radiation from said sample spots.
51. The device of item 50, wherein means for analyzing an event on the at least two sample spots associated with the biomolecule by detecting electromagnetic radiation from the at least two sample spots includes means for detecting electromagnetic radiation from an area on the support between the at least two sample spots.
52. The device of item 50 or 51 adapted for the sequence analysis of a single nucleic acid molecule.

### Detailed Description

The present disclosure relates to a support comprising a substrate and a plurality of sample spots on the support surface that are spatially separated from another. In certain embodiments, the support surface is formed by the substrate and the sample spots. In certain embodiments, the substrate forms a contiguous area in which the sample spots are distributed. An individual sample spot on the support surface is surrounded by the substrate, which differs from the sample spot, e.g., in respect of the material and/or the surface. Typically, the substrate is adapted for inhibiting and/or blocking adhesion of biomolecules such as polypeptides whereas the sample spots are adapted for allowing adhesion of desired biomolecules.

On at least two separate sample spots, a single biomolecule is immobilized. The term "single biomolecule" encompasses a single molecular entity such as a polypeptide or a complex consisting of a plurality of individual units, e.g., individual molecular entities wherein the individual units form together a functional biological moiety. In certain embodiments, a single biomolecule is immobilized on at least 2 and up to 10, 100, 1,000 or 10,000 separate sample spots, for example on 2, 3, 4, 5, 6, 7, 8, 9, or 10 separate sample spots, particularly on 2 separate sample spots. In certain embodiments, a biomolecule, e.g., a nucleic acid molecule with 1,000 nucleotides or more may be immobilized on a large number of separate sample spots, e.g., 100 or more sample spots.

In certain embodiments, the support is a substantially planar support, i.e., it does not comprise elevations or recesses of about 1000 nm or more or of about 100 nm or more. In further embodiments, the support is a structured support, e.g., a support comprising recesses such as wells that may have a volume of about 5 × 10⁻²⁴ liters to about 1 × 10⁻¹⁵ liter, or pillars of height of about 1 nm to 500 nm. In principle, the support may have any design, as long as a reaction space can be formed which enables the occurrence of a single molecule event on said at least two sample spots where the single biomolecules is immobilized.

In certain embodiments, the substrate is an optically transparent material, i.e., a material that is substantially transparent for electromagnetic radiation, e.g., radiation in the visible range and/or radiation in the near-infrared range. In certain embodiments, the substrate comprises a material having an absolute refractive index of at least 1.01, e.g., about 1.5 to about 3 in the visible range or about 1.5 to about 4 in the near-infrared range. In further embodiments, the substrate is an optically opaque material, e.g., a metal or semi-metal such as silicon.

In particular embodiments, the substrate comprises a non-electrically conductive material. Specific examples are glass, quartz, plastic, metal oxide-based materials, e.g., silicon dioxide-based materials such as glass, silica, or quartz, or composites comprising said materials. In further embodiments, the substrate comprises an electrically conductive material, e.g., an optically transparent material such as indium tin oxide.

Typically, the substrate has a thickness of about 10 µm to about 5 mm, particularly about 20 µm to about 2 mm.

In certain embodiments, the substrate is coated with a layer of diamond-like carbon and/or amorphous carbon (also designated as "carbon film") as described in copending application US 63/382,624, the content of which is herein incorporated by reference. The carbon film may be deposited on the substrate by physical vapor deposition, chemical vapor deposition (CVD) or atomic layer deposition (ALD) techniques. The carbon film may form a continuous layer on the surface of the support except for the area of the sample spots. In certain embodiments, the carbon film is fluorinated. Fluorine atoms may be introduced by known procedures, e.g., during DLC deposition by CVD or by exposure to a fluorine-containing plasma from fluorocarbon-based gases or the like (e.g., C₄F₈, CHF₃, NF₃, or SF₆).

In certain embodiments, the carbon film including a fluorinated carbon film has a thickness of about 0.3 nm to about 200 µm, particularly about 10 nm to about 100 µm, and more particularly about 1 µm to about 50 µm.

In certain embodiments, the surface of the substrate is coated with an organic passivating reagent, e.g., a polyethylene glycol containing reagent that inhibits and/or blocks adhesion of biomolecules such as proteins and/or nucleic acids.

The surface of the support comprises a plurality of sample spots that are spatially separated from another by the substrate surface. The sample spots are adapted for attachment of biomolecules. In certain embodiments, the support comprises a plurality of sample spots, e.g., at least 10, at least 100, at least 1,000, at least 10,000 sample spots, or at least 100,000 spots. In certain embodiments, the support may comprise up 10⁶ or 10⁹ or even more sample spots.

In certain embodiments, a sample spot comprises or consists of at least one electrically conductive material, e.g., a metal including a single metal or a combination of metals, e.g., an alloy or mixture of a plurality of different metals. For example, a metal that is capable of attachment to a sulfur containing moiety, e.g., in the form of a thiol or a disulfide, or a metal that is capable of attachment to a chelating moiety, e.g., a poly-histidine tag, is suitable. In certain embodiments, the metal has a positive electrochemical potential. Specific examples of suitable metals include but are not limited to Au, Cu, Ni, Pt, Pd, Rh, Ir, Os, Ru, and any combination thereof comprising at least two of said metals.

In certain embodiments, a sample spot comprises or consists of at least one at least one metal oxide including a single metal oxide or a combination of metal oxides. For example, a metal oxide that is capable of attachment to a phosphorus containing moiety, e.g., in the form of a phosphonic acid or phosphonic acid ester, or a metal oxide that is capable of attachment to a chelating moiety, e.g., a poly-histidine tag, is suitable. Specific examples of suitable metal oxides include TiO₂ and NiO.

In further embodiments, a sample spot comprises or consists of at least one non-electrically conductive material.

Sample spots may be prepared by vapour deposition of metals, which are vaporized on the support covered by a grid mask, which may be produced by electron beam lithography or equivalent technologies. The size of holes in the grid mask may correspond to the size of the spots on the support surface. Alternatively, the spots on the support may be prepared by site-specific deposition of nanoparticles, e.g., having a size of 2-10 nm, by precision pipetting of particles on the support, particularly on a support having a planar surface.

The sample spot may have a size that is suitable for the attachment of single biomolecules as defined herein. In particular embodiments, a sample spot has a diameter of about 1 nm to about 30 nm, particularly a diameter of about 2 nm to about 20 nm.

In certain embodiments, the sample spot is a separate object on the surface of the substrate. In certain embodiments, the sample spot has a lower face proximal to the substrate and an upper face distal to the substrate, wherein the distance between the lower and upper face defines the height of the sample spot. In particular embodiments, the height is about 50 pm to about 500 nm, particularly about 100 pm to about 20 nm, and more particularly about 500 pm to about 10 nm, e.g., about 2 nm.

According to the present disclosure, the size and configuration of sample spots on the support surface is selected such that a single biomolecule is immobilized on at least two separate spots.

In certain embodiments, individual sample spots are distributed substantially evenly on the support surface. In those embodiments, the distance between adjacent sample spots is about 1 nm to about 500 nm, e.g., about 2 nm to about 20 nm, or about 20 nm to about 500 nm, depending on the size of the biomolecule to be immobilized.

In certain embodiments, the sample spots are distributed unevenly on the support surface. In those embodiments, the sample spots may be distributed in groups of several spots, e.g., about 2 to about 10 spots or about 2 to about 4 spots on the support surface, wherein the distance between adjacent sample spots in different groups within a group is greater than the distance between adjacent sample spots in different groups. In certain embodiments, the distance between adjacent sample spots within a group is about 1 nm to about 50 nm, e.g., about 2 nm to about 20 nm. The groups of sample spots may be distributed in an even pattern, or in an uneven pattern on the support surface.

The sample spots within a group may be separated from the sample spots of an adjacent group by a distance which excludes the immobilization of a biomolecule to sample spots belonging to different groups. In certain embodiments, the distance between adjacent sample spots in different groups is about 2- to about 2000-times greater, e.g., about 5- to about 1000-times or about 10- to about 100-times greater than the distance between adjacent sample spots in the same group.

The sample spots on the support are adapted for the attachment of a biomolecule such that a single biomolecule is immobilized on at least two separate sample spots, e.g., by a covalent or non-covalent attachment. The biomolecule may be selected from polypeptides, nucleic acids, carbohydrates, and any combination thereof, e.g., a glycosylated polypeptide, or a ribonucleoprotein. In certain embodiments, the biomolecule is a complex consisting of several individual units, e.g., several polypeptide units, or several polypeptide and nucleic acid units.

In specific embodiments, the biomolecule is a nucleic acid-polymerizing enzyme, particularly a DNA polymerase or an RNA polymerase, or a nucleic acid-polymerizing molecule complex, particularly a DNA- or RNA-polymerizing complex comprising a nucleic acid-polymerizing enzyme and a nucleic acid molecule. In particular embodiments, the biomolecule is a DNA polymerase having a DNA binding cleft, particularly a Family A DNA polymerase including, but not limited to a Klenow, Taq or T7 DNA polymerase or any genetically modified version thereof, or a Family B polymerase including, but not limited to a therminator, Phi29, RB-69 or T4 DNA polymerase or any genetically modified version thereof. Reference is made in this context to US 7,745,116 B2, supra.

In further embodiments, the biomolecule is a nucleic acid-degrading enzyme, particularly an exonuclease, or a nucleic acid-degrading molecule complex, particularly a DNA- or RNA-degrading molecule complex comprising a nucleic acid-degrading enzyme and a nucleic acid molecule.

In still further embodiments, the biomolecule is a gene editing enzyme, particularly a Cas nuclease such as a Cas3, Cas9, Cas10, or Cas12 nuclease or any genetically modified version thereof, e.g., a Cas nickase, or a gene editing complex comprising a gene editing enzyme and nucleic acid molecule, e.g., a guide RNA and/or a target nucleic acid.

In still further embodiments, the biomolecule is a nucleic acid molecule, particularly a nucleic acid molecule as described in detail below.

In certain embodiments, the biomolecule comprises at least 2 anchors attached to at least 2 separate sample spots. e.g., 2, 3 or 4 anchors, attached to at least 2, e.g., 2, 3, or 4 separate sample spots.

In certain embodiments, the biomolecule is directly attached to the sample spots, e.g., by providing a biomolecule having anchors suitable for a direct covalent or non-covalent attachment to the surface of the sample spots, e.g., to an inorganic surface of the sample spots. In those embodiments, the anchors may comprise the reaction product of a sulfur-containing group such as a thiol group -SH, a substituted thiol group -SR, wherein R is an organic residue, e.g., a C₁-C₄ alkyl group or a disulfide group -S-S-, or a phosphorus-containing group with a metal or metal oxide surface on the sample spots. Alternatively, the anchors may comprise the reaction products of silane groups with a metal oxide, e.g., silica surface, or the reaction product of a poly(histidine) tag with a Ni or NiO surface.

In certain embodiments, the biomolecule is indirectly attached to the sample spots, e.g., by a non-covalent linkage to a coating, e.g., an organic coating on the surface of the sample spot. In those embodiments a biomolecule may be provided that has anchors comprising an attachment amino acid such as cysteine, a modified phenylalanine, histidine, or glutamine, or an attachment tag, e.g., a biotin, hapten, poly(histidine) tag or carbohydrate group, capable of forming a linkage with a complementary moiety of a coating attached to the surface of the sample spots, e.g., streptavidin, antibody, lectin, etc.

In certain embodiments, a biomolecule may be provided that has bio-orthogonal groups, i.e., groups not occurring in biomolecules such as an azide group or an alkyne group, e.g., a terminal or strained alkyne group such as cyclooctyne. Such bio-orthogonal groups are capable of forming covalent linkages with complementary bio-orthogonal groups attached to the surface of the sample spots. In those embodiments, the anchors may comprise the reaction product of a coupling reaction between 2 bio-orthogonal reactive groups. In certain embodiments, the coupling reaction is a Click reaction, e.g., a reaction between an azide group and an alkyne group. In certain embodiments, the anchors comprise a triazole group.

The support of the present disclosure is suitable for analyzing an event occurring on a sample spot, wherein the event is associated with the emission of an electromagnetic radiation from the sample spot. In particular embodiments, the event encompasses a reaction of a biomolecule which is associated with emission of a characteristic electromagnetic radiation. In particular embodiments, the event is a single molecule event. In certain embodiments, analyzing the event comprises directing electromagnetic radiation from a primary irradiation source, e.g., a laser onto the support, in order to cause excitation of luminescent, e.g., fluorescent groups associated with the event. In the following, the terms "luminescent" and "fluorescent" are used interchangeably.

In particular embodiments, the support of the present disclosure is suitable for analyzing an event, e.g., a single molecule event occurring on the at least one sample spot, particularly for separately analyzing a plurality of single molecule events each occurring on at least one sample spot and even more particularly for separately analyzing a plurality of single molecule events is analyzed in parallel. In particular embodiments, the single molecule event comprises a nucleic acid sequence determination.

A further aspect relates to a method for analyzing an event comprising:
(i) providing the above a support, wherein a single biomolecule is immobilized on at least two separate sample spots of the support, and
(ii) analyzing an event associated with said single biomolecule, by detecting electromagnetic radiation from said sample spots.

In particular embodiments, the event is a single molecule event, and the biomolecule is a single biomolecule.

In particular embodiments, the single molecule event comprises the sequence analysis of a single nucleic acid molecule.

A further aspect relates to a device adapted for analyzing an event comprising:
(i) the above support wherein a single biomolecule is immobilized on at least two separate sample spots of the support,
(ii) means for illuminating the at least two separate sample spots on the support with radiation, and
(iii) means for analyzing an event on said at least two sample spots by detecting electromagnetic radiation from said sample spots.

In particular embodiments, the event is a single molecule event, and the biomolecule is a single biomolecule.

In particular embodiments, the device is adapted for the sequence analysis of a single nucleic acid molecule.

Methods and devices for analyzing single molecule events are e.g., disclosed in WO 2002/097406, WO 2003/052137, WO 2006/013110, WO 2013/131888, WO 2015/104245, WO 2017/001407, and WO 2018/104301, the contents of which are herein incorporated by reference.

For the analysis of a single molecule event, the biomolecules are located at the sample spots on the support. There they are in contact with a sample liquid, which contains the free reaction partners. Thereby, one or more reaction spaces are defined. Particularly at least 100, at least 1000, or at least 10 000, and up to more than 10⁶ molecules may be analysed on a single support, e.g., a single planar support.

A nucleic acid molecule whose sequence is to be determined may be selected, for example, from DNA molecules such as genomic DNA fragments, cDNA molecules, plasmids, etc., or else from RNA molecules such as mRNA molecules. The nucleic acid molecule may originate from genomic or expression libraries, generated from cells or organisms, e.g., eukaryotic or prokaryotic cells or organisms. This allows parallel sequencing of a plurality of different nucleic acid template molecules, e.g., at least 10, 100, 1.000 or 10.000 and up to 100.000, 10⁶ or 10⁷ or even more different nucleic acid molecules.

The nucleic acid molecules to be sequenced may be single-stranded nucleic acid molecules in a linear or circular form, e.g., in a covalently linked circular form. In order to obtain a circular nucleic acid template, a linear nucleic acid molecule may be subjected to a circularization procedure and optionally a strand-separation procedure during sample preparation. Circularization may be effected by ligation according to known protocols, e.g., using DNA or RNA ligases. In some embodiments, an adaptor and/or identifier molecule, i.e., a nucleic acid molecule of known sequence, may be coupled to the nucleic acid molecule.

The sequence determination may comprise nucleic acid elongation and/or nucleic acid degradation. The sequencing process includes one or more sequencing cycles.

The nucleic acid-synthesizing enzyme molecules are capable of elongating a primer annealed to a nucleic acid template molecule. Primer elongation may be carried out by progressively incorporating individual nucleotide building blocks at the 3'-terminus of a growing nucleic acid chain, wherein a nucleic acid molecule complementary to the sequence of the circular nucleic acid template is generated. The nucleic acid-synthesizing enzymes are selected from polymerases capable of a template specific nucleic acid polymerization, preferably from DNA polymerases and RNA polymerases, e.g., natural or modified polymerases, including thermostable DNA polymerases, or reverse transcriptases.

Specific examples of suitable DNA polymerases include Taq polymerases, exonuclease-deficient Taq polymerases, E. coli DNA polymerase I, Klenow fragment, reverse transcriptase, Φ29-related polymerases including wild-type Φ29 polymerase and derivatives of such polymerases, such as exonuclease-deficient forms, T7 DNA polymerase, T5 DNA polymerase, an RB69 polymerase and others.

Nucleic acid-degrading enzyme molecules are capable of progressively cleaving off individual nucleotide building blocks from a nucleic acid molecule. Preferably exonucleases, more preferably single-strand exonucleases which degrade in the 3' >5' direction or in the 5'->3' direction are used. Exonucleases which are particularly preferably used are 3'→5' exonucleases such as E. coli exonuclease I and E. coli exonuclease III, and 5'→3' exonucleases such as T7 exonuclease, E. coli exonuclease II and E. coli exonuclease VIII. Further, the exonuclease activities of various polymerases, e.g., the Klenow fragment, Taq polymerase or T4 polymerase may be used.

The nucleic acid-synthesizing enzyme molecules are contacted with a linear or circular nucleic acid template molecule, e.g., a single-stranded DNA or RNA molecule, and a primer molecule annealed to the nucleic acid template molecule or capable of annealing thereto. The primer molecule is preferably a single-stranded nucleic acid or nucleic acid analogue molecule having a free 3'-end which can be extended by an enzymatic reaction catalyzed by the immobilized nucleic acid-synthesizing enzyme molecules. The length of the primer molecule is selected to allow effective annealing to the template under reaction conditions. Usually, the length of the primer molecule is at least 8, at least 10, at least 12 or at least 15 nucleotides and e.g., up to 20, 25, 50 or 100 nucleotides, or even higher. In some embodiments, the primer is resistant against digestion by nucleic acid-degrading enzyme molecules, e.g., by incorporating nucleotide analogue building blocks and/or linkages between nucleotide building blocks, which are stable against degradation. In other embodiments, the primer is sensitive against digestion by nucleic acid-degrading enzyme molecules.

The sequence of the primer is selected in that it effectively anneals under reaction conditions to the template molecule. For instance, the primer may be a universal degenerated primer capable of statistically annealing to unknown nucleic acid sequences. In other embodiments, the primer may be capable of annealing to a known sequence portion of the nucleic acid template molecule. In this embodiment, a known adaptor and/or identifier sequence may be incorporated into the nucleic acid template molecule. The primer may be unlabelled or comprise fluorescent labelling groups.

Further, the presence of nucleotide building blocks carrying at least one fluorescent labelling group is required. Preferably, each different nucleotide building block (A, G, C, T/U) contains a different luminescent, e.g., fluorescent labelling group.

The luminescent, e.g., fluorescent labelling groups may be selected from known fluorescent labelling groups used for labelling biopolymers, particularly nucleic acids, such as, for example, fluorescein dyes, rhodamines, oxazines, for example Evoblue or Gnothis Blue, phycoerythrin, Cy3, Cy5, IR dyes or derivatives thereof, etc.

The nucleotide building blocks may carry (i) a fluorescence labelling group which remains with the building block when the building block is incorporated into a nucleic acid molecule during a primer elongation catalyzed by a nucleic acid-synthesizing enzyme molecule, and/or (ii) a fluorescence labelling group which is cleaved off from the building block when the building block is incorporated into a nucleic acid molecule during a primer elongation catalyzed by a nucleic acid-synthesizing enzyme molecule. Fluorescence labelling groups remaining with the building block are preferably attached to the α-phosphate group, to the sugar and/or to the nucleobase group.

In particular embodiments, fluorescence labelling groups remaining with the building block are attached to the nucleobase, e.g., via a linker which may have a chain-length of up to 15, preferably of 10-12 carbon atoms, optionally including heteroatoms, e.g., N, O or S atoms. Fluorescence labelling groups which are cleaved off when the building block is incorporated into a nucleic acid molecule may be attached to a terminal phosphate group, e.g., of a polyphosphate building block including, but not limited to a hexa-, penta-, tetra- or triphosphate building block such as the γ-phosphate group of a triphosphate building block. In certain embodiments, building blocks are selected which contain both (i) a fluorescence labelling group remaining after incorporation and (ii) a fluorescence labelling group cleaved off during incorporation. In this case, fluorescence groups capable of interacting with each other, e.g., by quenching and/or energy transfer, may be selected.

The nucleic acid molecules to be sequenced will contain fluorescent labelling groups in case the nucleic acid molecule is subjected to direct sequencing using a nucleic acid-degrading enzyme molecule. On the other hand, the nucleic acid molecule to be sequenced may not contain fluorescent labelling groups, if the nucleic acid molecule is used as a template in a primer elongation.

The sequencing procedure may involve a step of generating nucleic acid molecules having incorporated nucleotide building blocks in a primer elongation catalyzed by the nucleic acid-synthesizing enzyme molecules and/or a second step of cleaving off individual nucleotide building blocks from the generated nucleic acid molecules catalyzed by nucleic acid-degrading enzyme molecules. Dependent on the type of luminescence, e.g., fluorescence labels, nucleic acid sequence determination may be carried out during primer elongation and/or during degradation.

Sequence determination during the primer elongation involves the use of nucleotide building blocks carrying a fluorescence labelling group which is cleaved off from the building block when it is incorporated into a nucleic acid molecule. In this case, a time-dependent fluorescence change caused by cleaving off the fluorescence labelling group from the nucleotide building block may be determined. Sequence determination during nucleic acid degradation involves the use of a nucleotide building block, which carries a fluorescence-labelling group which remains with the building block when it is incorporated into a nucleic acid molecule. Progressive cleavage of individual nucleotide building blocks from the nucleic acid molecules causes a time-dependent change of fluorescence when the labelled nucleotide building block is liberated from the nucleic acid molecule. In certain embodiments, it is also possible to carry out a sequence determination during elongation and degradation, i.e., when using nucleotide building blocks, which both carry a fluorescence labelling group remaining with the building block and a fluorescence labelling group which is cleaved off from the building block when the building block is incorporated into a nucleic acid molecule. In this embodiment, both fluorescent groups may be the same or different.

In some embodiments, the method involves one or more cycles of nucleic acid-synthesis and nucleic acid-degradation in order to determine the base sequence of a nucleic acid molecule template. The nucleic acid synthesis involves an elongation of the primer annealed to the nucleic acid template molecule catalyzed by the nucleic acid-synthesizing enzyme molecule, wherein a nucleic acid molecule complementary to the sequence of the nucleic acid template is generated. In the next step, the generated nucleic acid molecule is degraded by a nucleic acid-degrading enzyme molecule.

When a nucleotide building block is incorporated into an elongated nucleic acid molecule, a time dependent change in the fluorescence may occur, which can be detected as indicated above. Preferably, the incorporation of the nucleotide building blocks into the elongated nucleic acid molecule is associated with a detectable increase in the fluorescence, preferably with a transient increase in the fluorescence. For example, nucleotide building blocks may be used which carry a fluorescent labelling group on the portion of the molecule which is cleaved off when the building block is incorporated into the primer, e.g., on the γ-phosphate group.

When a nucleotide building block is cleaved off from the synthesized nucleic acid molecule, a time-dependent change of fluorescence may be determined due to the interaction of fluorescent labelling groups incorporated in nucleic acid strands with neighbouring groups, for example with chemical groups of the nucleic acids, in particular nucleobases such as, for example, G, or/and neighbouring fluorescent labelling groups, and these interactions leading to a change in fluorescence, in particular in fluorescence intensity, compared to the fluorescent labelling groups in "isolated" form, owing to quenching processes or/and energy transfer processes. The removal by cleavage of individual nucleotide building blocks alters the overall fluorescence, for example the fluorescence intensity of an immobilized nucleic acid strand, and this change is a function of the removal by cleavage of individual nucleotide building blocks, i.e., a function of time.

In certain embodiments, association of a labelled nucleotide with the biomolecule complex is detected by measuring polarisation of the emitted photons. The polarisation of excited states' photons is changed by the rotational movement of the light emitting nucleotide labels and can be used for identifying free moving contra bound labelled nucleotides in the polymerisation process.

This time-dependent change in fluorescence during elongation and/or degradation may be recorded in parallel for a multiplicity of nucleic acid molecules and correlated with the base sequence of the individual nucleic acid strands. Preference is given to using those fluorescent labelling groups which, when incorporated in the nucleic acid strand, are, at least partially, quenched so that the fluorescence intensity is increased after the nucleotide building block containing the labelling group or a neighbouring building block causing quenching has been removed by cleavage.

During incorporation and/or removal of individual nucleotide building blocks, it is possible to measure a change in fluorescence intensity of the nucleic acid strand or/and the incorporated or cleaved-off nucleotide building block, owing to quenching processes or energy transfer processes. This change in fluorescence intensity with time depends on the base sequence of the nucleic acid strand studied and can therefore be correlated with the sequence.

The complete sequence of the nucleic acid molecule may be determined by using a mixture of nucleotide building blocks, labelled on all four different bases, for example on A, G, C and T, or on combinations of two or three different bases. It is possible, where appropriate, to attach to the nucleic acid strand to be studied also a "sequence identifier", i.e., a labelled nucleic acid of known sequence, for example by enzymatic reaction using ligase or/and terminal transferase, so that at the start of sequencing initially a known fluorescence pattern and only thereafter the fluorescence pattern corresponding to the unknown sequence to be studied is obtained.

The detection comprises irradiating light into the support, preferably by means of a laser, or by another suitable light source, in order to cause excitation of the fluorescent labelling groups. It is possible, in this connection, to use one or more laser beams, for example an expanded laser beam, having a cross section of approx. 1-20 mm, and/or multiple laser beams. The detection preferably comprises a multipoint fluorescence excitation by lasers, for example a dot matrix of laser dots generated via diffraction optics (cf. WO 2002/097406) or a quantum well laser.

Fluorescence emission of a plurality of nucleic acid strands may be detected in parallel using a detector matrix which comprises, for example, an electronic detector matrix, for example a CCD camera, a CMOS detector matrix, e.g., a CMOS camera, or an avalanche photodiode matrix. The detection may be carried out in such a way that fluorescence excitation and detection are carried out in parallel on a part or all nucleic acid strands studied. Preference is given to carrying out the detection on fluorescence light which is emitted essentially orthogonally from the support surface through the reaction space or through the support body.

The detection may be carried out, for example, by means of a single molecule detection, for example by fluorescence correlation spectroscopy, which involves exposing a very small, preferably confocal, volume element, for example from 10⁻²¹ to 10⁻¹⁰ I, to the excitation light of a laser, or another suitable light source, which light excites the receptors present in this measuring volume so that the latter emit fluorescence light, the fluorescence light emitted from said measuring volume being measured by means of a photodetector and the change in the measured emission with time being correlated with the concentration of the analyte, so that it is possible to identify, at an appropriately high dilution, individual molecules in said measuring volume. Details of the procedure and of the apparatus used for detection can be found in the disclosure of EP 0 679 251, the content of which is herein incorporated by reference. The confocal determination of single molecules is furthermore described in Rigler and Mets (Soc. Photo-Opt. Instrum. Eng. 1921 (1993), 239 ff.) and Mets and Rigler (J. Fluoresc. 4 (1994) 259-264), the contents of which are herein incorporated by reference.

Alternatively, or additionally, detection may also be carried out by way of time-resolved decay measurement, called "time gating", as described, for example, by Rigler et al., "Picosecond Single Photon Fluorescence Spectroscopy of Nucleic Acids", in: "Ultrafast Phenomena", D.H. Auston, Ed., Springer 1984, the content of which is herein incorporated by reference. Here, the fluorescent molecules are excited in a measuring volume followed by, e.g., at a time interval of ≥ 100 ps, opening a detection interval on the photodetector. In this way it is possible to keep background signals generated by Raman effects sufficiently low so as to enable single molecules to be detected in an essentially interference-free manner.

In certain embodiments of the present disclosure, the single biomolecule to be attached to multiple sample spots is a nucleic acid molecule, e.g., a single- or double-stranded nucleic acid molecule. In particular embodiments, the single biomolecule is a single DNA molecule or a single RNA molecule.

In certain embodiments, the nucleic acid molecule has a length of at least about 10 nucleotides, at least about 100 nucleotides, at least about 1,000 nucleotides, e.g., about 1,000 to about 100,000, particularly about 10,000 to about 50,000 nucleotides, e.g., deoxyribonucleotide building blocks, ribonucleotide building blocks and/or nucleotide analogue building blocks.

The typical length of a nucleic acid molecule, e.g., a DNA or RNA molecule is the length of a human gene, that is on the average, approximately 15 000 nucleotides. One base has a length of approximately 0.3 nm. Nucleic acid molecules having shorter or larger lengths may be used for certain applications.

In those embodiments, methods and devices for a parallel read-out of the sequence of several different sections of a single DNA or RNA molecule are provided.

The nucleic acid molecules, e.g., the DNA or RNA molecules comprise at least one anchor, particularly at least two or more anchors for immobilization to a support having a surface comprising a plurality of sample spots, e.g., metal spots, as described above. In particular embodiments, only one anchor is bound to one sample spot.

In certain embodiments, the anchors are suitable for directly or indirectly attaching the nucleic acid molecule to the sample spots, as described above. In those embodiments, the biomolecule may have bio-orthogonal groups as described above.

In particular embodiments, an anchor comprises a capture probe comprising a nucleic acid sequence complementary to a sequence portion of the single nucleic acid molecule to be immobilized on the sample spots. The capture probe forms a double-stranded hybrid with the single nucleic acid molecule which is thereby immobilized to the sample spot to which the probe is attached. The length of the complementary sequence in the capture probe is sufficient to allow a stable hybrid formation when analyzing the single molecule event.

In certain embodiments, the length of the complementary sequence in the capture probe is at least about 10, at least about 12, at least about 14, and at least about 16 nucleotides including nucleotide analogues. In certain embodiments, the length of the complementary sequence in the capture probe is up about 100, up to about 80, up to about 60, and up to about 40 nucleotides including nucleotide analogues. For the immobilization of single nucleic acid molecule at least 2, e.g., 2, 3, 4, 5, 6, 7, 8, 9,10 or even more different capture probes may be used. The different capture probes are capable of hybridizing to different sections of the single nucleic acid molecule. The sections to which the capture probes can hybridize may be selected specifically or randomly.

In certain embodiments, the immobilized single nucleic acid molecule serves as a template strand for a nucleic acid polymerization process where a new nucleic acid strand complementary to the template strand is generated by primer elongation. In those embodiments, one or more or all anchors possess the functionality of a primer, i.e., being a 3' starting point for a nucleic acid polymerizing enzyme, e.g., a DNA polymerase, an RNA polymerase or a reverse transcriptase, as described above. For this purpose, the anchors may have a capture probe sequence with a free 3'-end accessible for a nucleic acid polymerizing enzyme. The polymerization comprises incorporation of individual nucleotide building blocks into the new nucleic acid strand. This incorporation event results in the emission of electromagnetic radiation, which can be detected as described above.

In embodiments, where the immobilized single nucleic acid molecule is a template strand for a nucleic acid polymerization process, the nucleic acid polymerizing enzyme is typically present as free molecule, i.e., not immobilized to a sample spot on the support, in the reaction medium. Further, the reaction medium contains luminescent, e.g., fluorescent nucleotide building blocks for incorporation into the new nucleic acid strand generated by primer elongation.

In certain embodiments, detection of the emitted electromagnetic radiation is made with approximately equal sensitivity over the whole surface of the support, i.e., the area of the sample spots, and the area between the sample spots. In those embodiments, the optical focus of detection of emitted electromagnetic radiation is not directed solely on the emitted electromagnetic radiation stemming from the areas of the individual sample spots but also to the areas in between the sample spots.

The primary radiation source may be directed to the support such that a substantially even primary radiation field or an even primary radiation field is generated at the surface so that all molecules located at or near the surface are illuminated. The intensity of the primary radiation field is typically declining proportional by r⁻² (where r is the distance to the surface in the orthogonal direction) in the direction orthogonal to the surface and, consequently, the primary radiation field is largest close to the surface.

The immobilization of a nucleic acid molecule, e.g., a DNA or RNA molecule by several anchors to multiple sample spots on the support provides an approximately horizontal positioning of the whole nucleic molecule with respect to the support surface. In between individual attachment positions, the nucleic acid molecule may be curved in all three dimensions to a certain limited extent. This allows an efficient and sensitive detection of the emitted electromagnetic radiation caused by incorporation of nucleotide building blocks throughout the whole length of the immobilized nucleic acid molecule.

The read-out of the sequence of a single DNA or RNA molecule can be made in parallel for individual sections thereof defined by the individual anchors. The number of anchors used for a nucleic acid molecule correlates with the degree of read-out parallelism that can be obtained for a certain molecule. The speed of read out of the sequence of an individual nucleic acid molecule, e.g., a DNA or RNA molecule is approximately proportional to the number of anchors. Hence, using a large number of anchors, e.g., one anchor per about 50 nucleotides to about 1,000 nucleotides or more a very large read-out speed of the sequencing of a DNA or RNA molecule can be obtained.

Further, the present disclosure is explained in detail by reference to the following specific embodiments.

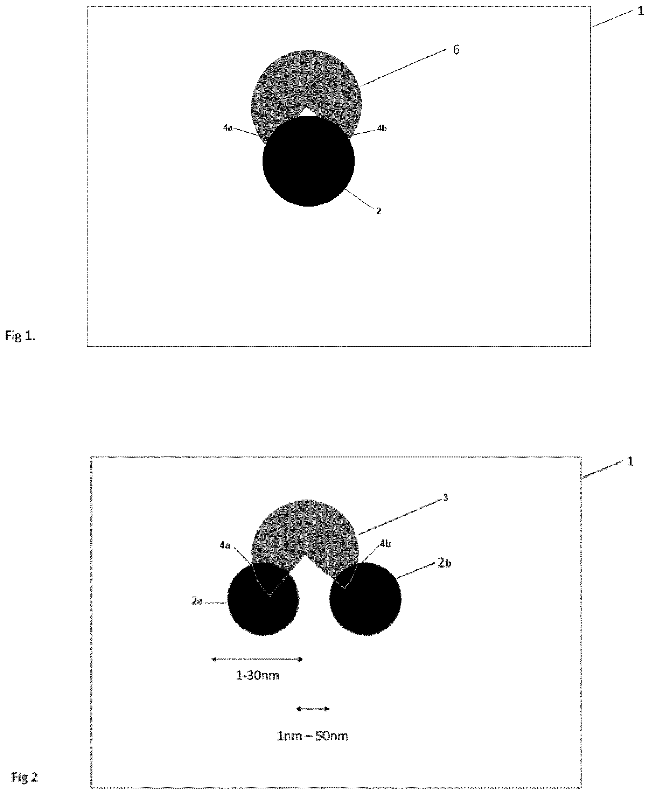

Fig. 1 shows an embodiment of the prior art. A support (1) comprises a sample spot (2) onto which a single biomolecule (3), e.g., a DNA polymerase molecule, is immobilized via anchors (4a, 4b). Both anchors are immobilized to the same sample spot.
Fig. 2 shows an embodiment of the present disclosure. A support (1) comprises two sample spots (2a, 2b) onto which a single biomolecule (3), e.g., a DNA polymerase molecule, is immobilized via anchors (4a, 4b). Each anchor is immobilized to a separate sample spot. The diameter of a sample spot is e.g., in the range of 1-30 nm. The distance between adjacent spots is e.g., in the range of 1-50 nm.

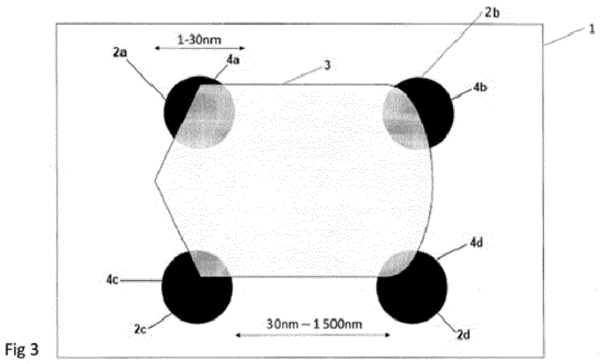

Fig. 3 shows an embodiment of the present disclosure. A support (1) comprises four sample spots (2a, 2b, 2c, 2d) onto which a single biomolecule (3), e.g., nucleic acid-polymerizing complex or a gene editing complex consisting of a plurality of individual units, is immobilized via anchors (4a, 4b, 4c, 4d). Each anchor is immobilized to a separate binding. The diameter of a sample spot is e.g., in the range of 1-30 nm. The distance between adjacent spots is e.g., in the range of 30 nm-1,500 nm.

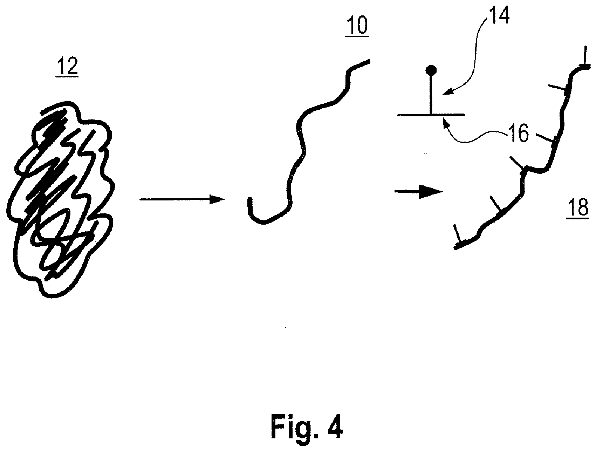

Fig. 4 shows the preparation of a single-stranded DNA or RNA molecule (10) from a sample (12). Suitable samples include but are not limited to biological samples such as blood, animal, e.g., human tissue, hair, and cultured cells. Anchors (14) comprising capture probe sequences (16) are attached to the DNA or RNA molecule. In certain embodiments, the capture probe sequences bind specifically to certain sections of the DNA or RNA molecule. Alternatively, the capture probe sequences bind stochastically, non-specifically to the DNA or RNA molecule. Thereby, a DNA or RNA molecule with anchors bound thereto (18) is obtained.

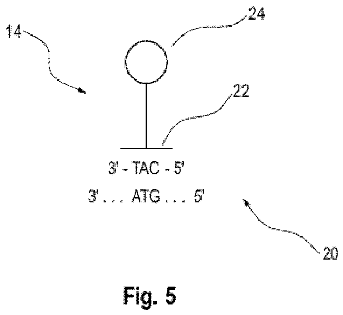

Fig. 5 shows an exemplary anchor (14) bound to a DNA or RNA molecule (20). The anchor (14) comprises a capture probe (22) having the sequence 5'-TAC-3' complementary to a certain section 5'- ... GTA ...3' of the DNA or RNA molecule (20) and an accessible 3'-end and an attachment group (24). The capture probe (22) binds to the DNA or RNA molecule (20) and the attachment group (24) binds to a sample spot.

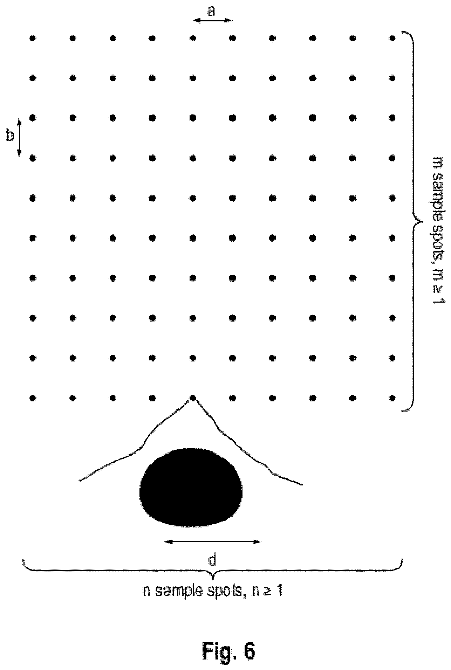

Fig. 6 shows an exemplary support comprising a surface comprising m x n sample spots having a diameter d separated from each other by the distance a or b, respectively. The parameters a, b, d, and m x n are as described herein above.

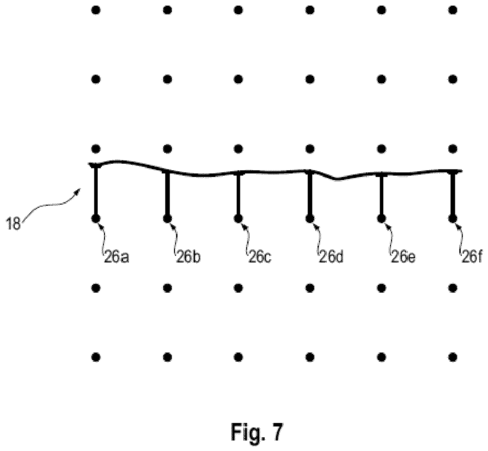

Fig. 7 shows an exemplary DNA or RNA molecule with anchors bound thereto (18) immobilized on a plurality of sample spots (26a, 26b, 26c, 26d, 26e, 26f).

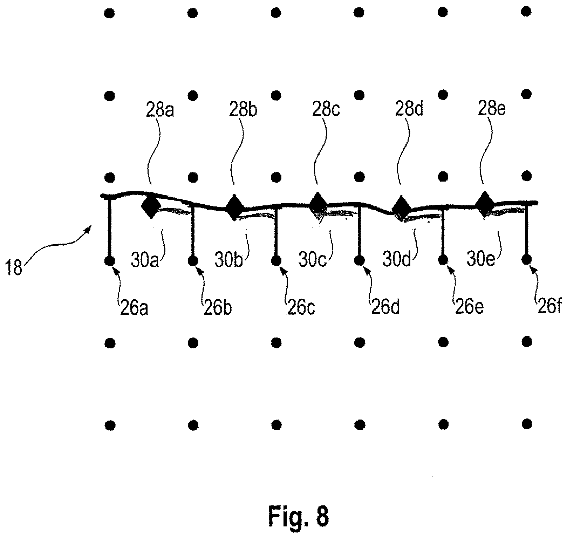

Fig. 8 is a schematic depiction of a polymerization process on the DNA or RNA molecule (18) of Fig. 7. Several polymerase molecules (28a, 28b, 28c, 28d, 28e) catalyse simultaneous polymerization reactions at mutually different sections of the DNA or RNA molecule (18). Thereby, complementary nucleic acid strands (30a, 30b, 30c, 30d, 30e) are generated. The polymerase molecules may be added to an immobilized DNA or RNA molecule. The polymerase molecules are freely diffusing in the medium surrounding the immobilized DNA or RNA molecule, until bound to the 3'-end of a capture probe. Also diffusing freely in the solution surrounding the DNA or RNA molecule are nucleotides of all four base categories A, T/U, G, and C. When a polymerase molecule attaches to the 3'- end of a capture probe, polymerization starts and continues until the polymerase reaches the next capture probe.

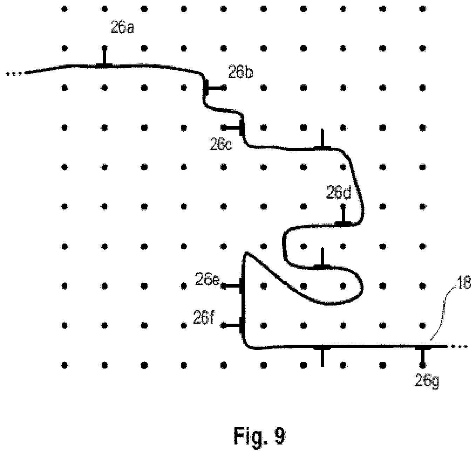

Fig. 9 shows a further example of a DNA or RNA molecule (18) attached to sample spots (26a, 26b, 26c, 26d, 26e, 26f, 26g) via anchors comprising attachment groups. For some applications it may not be necessary that all attachment groups are bound to a sample spot (not shown).

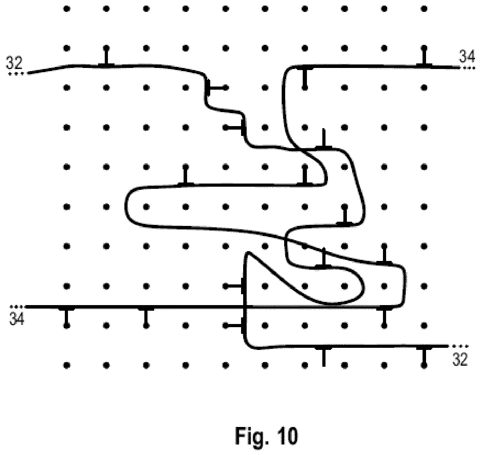

Fig. 10 shows several DNA or RNA molecules attached to the surface. The figure indicates this situation for the case of two molecules (32, 34). In certain embodiments, the number of molecules attached to the surface in one measurement is about 1,000, but can be 10,000, 100,000, 1,000,000, or even 100,000,000. For some applications only a few molecules may be desired, hence below 1,000, typically one, 10 or 100 molecules attached to the surface simultaneously. A partial overlap between different molecules may reduce accuracy in such overlapping regions only. In regions that do not overlap, the presence of many different molecules on the surface simultaneously does not negatively affect the accuracy.

## Claims

1. A support comprising a substrate and a plurality of sample spots on the surface of the support, wherein the sample spots are spatially separated from another, and wherein a biomolecule is immobilized on at least 2 separate sample spots.

2. The support of claim 1, which comprises at least 10, at least 100, at least 1,000, at least 10,000, at least 100,000, at least 1,000,000 or at least 10,000,000 sample spots.

3. The support of any one of the preceding claims, wherein the at least one sample spot has a diameter of about 1 nm to about 30 nm, particularly a diameter of about 2 nm to about 20 nm.

4. The support of any one of the preceding claims, wherein the sample spots are distributed substantially evenly on the support surface, wherein the distance between adjacent sample spots is particularly about 1 nm to about 500 nm, e.g., about 2 nm to about 20 nm, or about 20 nm to about 500 nm.

5. The support of any one of claims 1-3, wherein the sample spots are distributed unevenly on the support surface, wherein the sample spots are particularly distributed in groups of several spots, e.g., about 2 to about 10 spots or about 2 to about 4 spots on the support surface, wherein the distance between adjacent sample spots in different groups within a group is greater than the distance between adjacent sample spots in different groups.

6. The support of claim 5, wherein the distance between adjacent sample spots within a group is about 1 nm to about 50 nm, e.g., about 2 nm to about 20 nm, and/or wherein the distance between adjacent sample spots in different groups is about 2- to about 2000-times greater, e.g., about 5- to about 1000-times or about 10- to about 100-times greater than the distance between adjacent sample spots in the same group.

7. The support of any one of the preceding claims, wherein the biomolecule is selected from the group consisting of polypeptides, nucleic acids, carbohydrates, and any combination thereof,
wherein the biomolecule is particularly a nucleic acid-polymerizing enzyme, particularly a DNA polymerase or an RNA-polymerase, or a nucleic acid-polymerizing molecule complex, particularly a DNA- or RNA-polymerizing molecule complex comprising a nucleic acid-polymerizing enzyme and a nucleic acid molecule, or
wherein the biomolecule is particularly a gene editing enzyme, particularly a Cas nuclease such as a Cas9 nuclease or any genetically modified version thereof, or a gene editing complex comprising a gene editing enzyme and nucleic acid molecule, e.g., a guide RNA and/or a target nucleic acid, or wherein the biomolecule is particularly a nucleic acid molecule, e.g., a single-or double-stranded single- stranded DNA or RNA molecule, wherein the nucleic acid molecule has a length of at least about 10 nucleotides, at least about 100 nucleotides at least about 1,000 nucleotides, e.g., about 1,000 to about 100,000, particularly about 10,000 to about 50,000 nucleotides.

8. The support of any one of the preceding claims, wherein the biomolecule comprises at least 2 anchors attached to at least 2 separate sample spots.

9. Use of a support of any one of claims 1-8 for analyzing an event, particularly a single molecule event occurring on the at least two separate sample spots, wherein said event is associated with emission of an electromagnetic radiation.

10. Use of a support of any one of claims 1-8 for separately analyzing a plurality of events, particularly single molecule events each occurring on the at least two separate sample spots, wherein said events are associated with emission of an electromagnetic radiation, wherein said plurality of events is particularly analyzed in parallel.

11. The use of any one of claims 9-10, wherein the single molecule event comprises a nucleic acid sequence determination comprising:
- providing (i) a single nucleic acid molecule, (ii) a nucleic acid-synthesizing enzyme molecule and/or a nucleic acid degrading enzyme molecule immobilized on at least two separate sample spots and (iii) fluorescence labelled nucleotide building blocks in free form and/or incorporated into the nucleic acid molecule,
- conducting an enzymatic reaction, wherein nucleotide building blocks are incorporated into and/or cleaved off from said single nucleic acid molecule, and
- individually determining the base sequence of the nucleic acid molecule on the basis of the time-dependent fluorescence change, caused when nucleotide building blocks are incorporated into and/or cleaved off from said single nucleic acid molecule.

12. A method for analyzing an event, e.g., a single molecule event comprising:
(v) providing a support of any one of claims 1-8, wherein a single biomolecule is immobilized on at least two separate sample spots of the support, and
(vi) analyzing an event, associated with said biomolecule, particularly a single molecule event associated with said single biomolecule, by detecting electromagnetic radiation from said sample spots.

13. The method of claim 12, wherein the single molecule event comprises a sequence analysis of a single nucleic acid molecule.

14. A device for analyzing an event, e.g., a single molecule event comprising:
(vii) a support of any one of claims 1-8, wherein a single biomolecule is immobilized on at least two separate sample spots of the support,
(viii) means for illuminating the at least two separate sample spots on the support with radiation, and
(ix) means for analyzing an event on said at least two sample spots by detecting electromagnetic radiation from said sample spots.

15. The device of claim 14 adapted for the sequence analysis of a single nucleic acid molecule.
